# EUROPÄISCHE PATENTSCHRIFT

(11) **EP 0 589 214 B1**
(45) Veröffentlichungstag und Bekanntmachung des Hinweises auf die Patenterteilung: **13.12.1995**
(21) Anmeldenummer: 93113409.2
(22) Anmeldetag: 23.08.1993
(51) Int. Cl.: C25B 3/06, C07C 19/08, C07C 17/361, C07C 17/093, C07C 53/18, C07C 53/21, C07C 59/115, C07C 59/135

(54) **Verfahren zur Herstellung von perfluorierten Bromalkanen oder Ethergruppen enthaltenden perfluorierten Bromalkanen**
Process for preparing perfluorinated bromoalkanes or perfluorinated bromoalkanes containing ether groups
Procédé de préparation de bromoalcanes perfluorés pouvant présenter également des groupes éther

(30) Priorität: 23.09.1992 DE 4231744
(43) Veröffentlichungstag der Anmeldung: 30.03.1994
(73) Patentinhaber: HOECHST AKTIENGESELLSCHAFT, 65926 Frankfurt am Main (DE)
(72) Erfinder: Millauer, Hans, Dr., D-65760 Eschborn (DE)

(56) Entgegenhaltungen:
- EP-A- 0 200 908
- EP-A- 0 261 501
- EP-A- 0 295 582
- EP-A- 0 404 076
- FR-A- 1 404 744
- US-A- 3 274 081

## Beschreibung

Die Erfindung betrifft ein Verfahren zur Herstellung von perfluorierten Bromalkanen der allgemeinen Formel

X-(CF₂)p-Br (I),

worin X Wasserstoff oder Fluor bedeutet und p für eine ganze Zahl von 2 bis 10 steht,
oder von Ethergruppen enthaltenden perfluorierten Bromalkanen der allgemeinen Formel 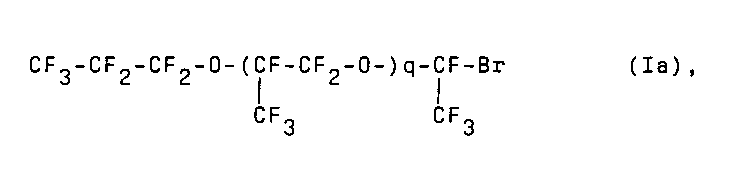
worin
q für Null oder eine ganze Zahl von 1 bis 4 steht.

Langkettige perfluorierte Bromalkane wie beispielsweise Perfluor-1-bromoctan oder Ethergruppen enthaltende perfluorierte Bromalkane wie beispielsweise Perfluor-2-brom-5-methyl-3,6-dioxanonan besitzen ein beträchtliches Lösevermögen für Sauerstoff und Kohlendioxid und werden deshalb auf dem medizinischen Sektor als Blutersatzstoff sowie als Röntgenkontrastmittel eingesetzt.
Kurzkettige, 1 H-Atom enthaltende perfluorierte Bromalkane, wie beispielsweise H-CF₂-CF₂-Br sind als Feuerlöschmittel mit niedrigem Ozonabbaupotential geeignet.

Perfluorierte Bromalkane bzw. Ethergruppen enthaltende perfluorierte Bromalkane können nach verschiedenen Verfahren hergestellt werden:
Gemäß der Arbeitsweise der DE-C-1 155 104 wird durch Bromierung von Trifluormethan im Gaszustand Trifluorbrommethan hergestellt.

Gemäß der Arbeitsweise der EP-A-0 295 582 werden durch Decarboxylieren von trockenen Salzen von perfluorierten Alkancarbonsäuren oder Oxa-alkancarbonsäuren in Gegenwart von Brom in einem aprotischen Lösemittel perfluorierte Bromalkane hergestellt.

Es war daher die Aufgabe gestellt, ein Verfahren zur Herstellung von perfluorierten Bromalkanen sowie von Ethergruppen enthaltenden perfluorierten Bromalkanen anzugeben, bei welchem Fluoralkancarbonsäuren sowie Ethergruppen enthal tende Fluoralkancarbonsäuren als Ausgangsstoffe umgesetzt werden. Die Ausgangsstoffe sollen mit hoher Ausbeute und guter Reinheit in das gewünschte Endprodukt umgewandelt werden. Das Verfahren soll auch kontinuierlich betrieben werden können.

Überraschenderweise konnte die Aufgabe dadurch gelöst werden, daß man eine perfluorierte Alkancarbonsäure der allgemeinen Formel

X-(CF₂)p-COOH (II),

worin X Wasserstoff oder Fluor bedeutet und p für eine ganze Zahl von 2 bis 10 steht,
oder eine Ethergruppen enthaltende Alkancarbonsäure der allgemeinen Formel 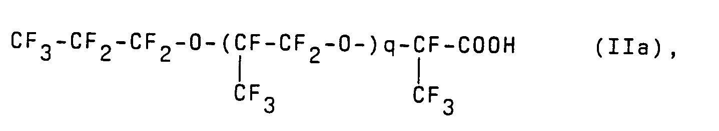
worin
q für Null oder eine ganze Zahl von 1 bis 4 steht,
in einem wäßrigen Elektrolyten in Gegenwart von Brom und einem aliphatischen Nitril einer elektrolytischen Decarboxylierung unterwirft.

Das erfindungsgemäße Verfahren kann wahlweise und bevorzugt dadurch gekennzeichnet sein, daß man
a) ein aliphatisches Nitril mit 1 bis 6 C-Atomen verwendet;
b) als aliphatisches Nitril Acetonitril, Propionitril oder Isobutyronitril verwendet;
c) als wäßrigen Elektrolyten ein Gemisch aus
   1 bis 40 Gew% Nitril
   1 bis 70 Gew% Alkancarbonsäure der Formel II oder IIa
   5 bis 100 mol% Alkalihydroxid, berechnet auf die Alkancarbonsäure
   50 bis 200 mol% Brom, berechnet auf die Alkancarbonsäure
   Rest Wasser
   elektrolysiert;
d) als Alkancarbonsäuren
   α) Omega-H-Perfluoralkansäuren der Formel II mit X = H:
      3-H-Perfluorpropionsäure, 5-H-Perfluorpentansäure,
      7-H-Perfluorheptansäure, 9-H-Perfluornonansäure;
   β) Perfluoralkansäuren der Formel II mit X = F:
      Perfluorpentansäure, Perfluorhexansäure, Perfluorheptansäure, Perfluoroctansäure, Perfluornonansäure, Perfluordecansäure oder Perfluorundekansäure;
   γ) Perfluorethercarbonsäuren der Formel IIa:
      Perfluor-2-methyl-3-oxahexansäure, Perfluor-2,5-dimethyl-3,6-dioxanonansäure, Perfluor-2,5,8-trimethyl-3,6,9-trioxadodecansäure oder Perfluor-2,5,8,11-tetramethyl-3,6,9,12-tetraoxapentadecansäure verwendet;
e) die Alkancarbonsäuren in neutralisierter oder teilweise neutralisierter Form einsetzt;
f) als Alkalihydroxid zur Neutralisation oder Teilneutralisation der Alkancarbonsäure Natriumhydroxid, Kaliumhydroxid oder Tetraalkylammoniumhydroxid einsetzt;
g) während der elektrolytischen Decarboxylierung Alkancarbonsäure in nichtneutralisierter Form in dem Maße nachdosiert, wie diese bei der elektrolytischen Decarboxylierung verbraucht wird;
h) das Brom portionsweise oder kontinuierlich während der elektrolytischen Decarboxylierung dem Elektrolyten zugibt;
i) eine Stromdichte von 20 bis 500, insbesondere von 50 bis 250 mA/cm² einstellt;
j) die elektrolytische Decarboxylierung bei Temperaturen zwischen -10°C und 50°C durchführt;
k) die elektrolytische Decarboxylierung in ungeteilten Becherglaszellen oder Durchflußzellen durchführt;
l) die elektrolytische Decarboxylierung in einer geteilten Elektrolysezelle durchführt, wobei der Anolyt- und Kathodenraum durch eine ionenselektive Membran oder ein Diaphragma voneinander getrennt sind;
m) die Alkancarbonsäure und das Brom in den Anolytraum gibt.

Bei der Verwendung einer geteilten Elektrolysezelle wird zweckmäßig die Alkansäure in neutralisierter Form eingesetzt. Bei Verwendung ungeteilter Elektrolysezellen sind Neutralisationsgrade zwischen 5 und 100 % zweckmäßiger.

Als Anodenmaterial werden Platin oder andere Edelmetalle, mit Platin oder anderen Edelmetallen beschichtete Metalle und glasartiger Kohlenstoff eingesetzt. Als Kathodenmaterial werden die als Anodenmaterial genannten Werkstoffe sowie Graphit oder Titan verwendet.

Überraschenderweise bilden sich die gewünschten Produkte der Formel I bzw. Ia nur dann, wenn man als Elektrolyt Gemische aus Wasser und Nitril verwendet. Dagegen werden keine Produkte der Formel I bzw. Ia erhalten, wenn man entweder nur Wasser oder nur ein Nitril als Elektrolyt einsetzt.

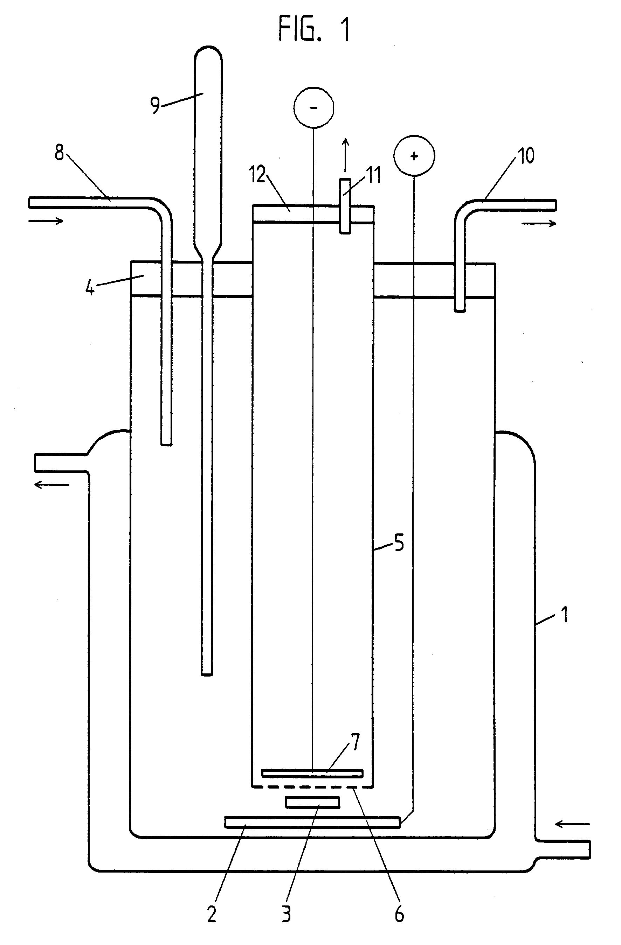

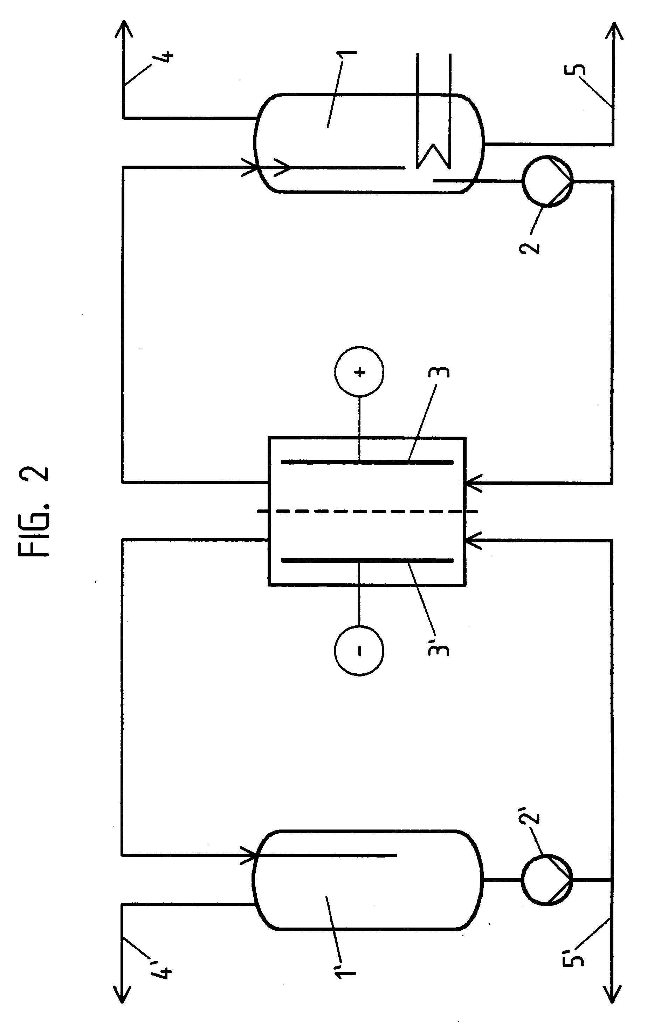

Die Erfindung wird anhand der Figuren 1 und 2 sowie der Beispiele näher erläutert:

### Beispiel 1

Im Beispiel 1 wird eine Becherglaszelle, wie in Figur 1 schematisch im senkrechten Schnitt dargestellt, benutzt. In einem mit einem äußeren Kühlmantel 1 versehenen, zylindrischen Glasgefäß mit einem Innendurchmesser von 100 mm und einer Höhe von 150 mm befindet sich auf dem Boden ein kreisförmiges Platinblech (Fläche ca. 35 cm²), welches als Anode 2 dient. Auf der Anode liegt ein Magnetrührstab 3 von 30 mm Länge. Die Zelle ist mit einem Stopfen 4 aus Polyethylen, welcher mit mehreren Bohrungen versehen ist, verschlossen. Durch die zentrale Bohrung wird ein Rohr 5 (Innendurchmesser 50 mm) aus Polyethylen geführt, das an seinem unteren Ende mit einer waagerecht liegenden, mittels eines Schraubverschlusses eingespannten Kationentauschermembran 6 (Nafion ® 427) verschlossen ist. Das Rohr 5 ist am Kopf mit einem Stopfen 12 aus Polyethylen verschlossen. In dem so gebildeten Kathodenraum befindet sich ein ebenfalls waagerecht liegendes Netz aus Chromnickelstahl (Fläche ca. 23 cm²), welches als Kathode 7 dient. Durch den Stopfen 4 wird außerdem ein Stickstoffeinleitungsrohr 8 sowie ein Thermometer 9 eingeführt. Durch die Ableitungen 10 und 11 entweichen gasförmige Reaktionsprodukte aus dem Anodenraum und dem Kathodenraum. Als Anolyt wird eine neutrale Lösung von 92,8 g (0,20 Mol) Perfluornonansäure (Formel II, X = F, p = 8) in 230 g 0,35 gew%iger wäßriger Natronlauge, 100 g Acetonitril und 24 g (0,30 Mol) Brom eingesetzt. Der Katholyt besteht aus 100 g 1 gew%iger wäßriger Natronlauge.
Die Elektrolyse erfolgt bei einer Temperatur von 20°C und einer Stromstärke von 1,8 Ampere bis zu einem Ladungsdurchgang von 0,25 Faraday, die Rührgeschwindigkeit des Magnetstabes beträgt 600 Upm. Nach der Elektrolyse wird das abgeschiedene Produkt im Scheidetrichter abgetrennt, mit Wasser gewaschen und über Natriumsulfat getrocknet. Man erhält 97,2 g Rohprodukt, welches nach gaschromatografischer Analyse 95,1 % Perfluor-1-bromoctan (Formel I, X = F, p = 8) enthält. Die daraus berechnete Produktausbeute beträgt 92,5 %; die Stromausbeute beträgt 80 %. Durch fraktionierte Destillation erhält man ein Produkt mit einem Siedepunkt von 140 bis 141°C.

### Beispiel 2

Das Beispiel 1 wird mit anderem Einsatzstoff nachgearbeitet. Ausgehend von 82,8 g (0,20 Mol) Perfluoroctansäure (Formel II, X = F, p = 7) und 24 g (0,30 Mol) Brom erhält man 84,2 g Perfluor-1-bromheptan (Formel I, X = F, p = 7) mit einer Reinheit von 93 %. Das entspricht einer Produktausbeute von 87,2 % und einer Stromausbeute von 72,5 %. Durch fraktionierte Destillation erhält man ein farbloses Produkt mit einem Siedepunkt von 121 - 122°C.

### Beispiel 3

Für das Beispiel 3 wird die in Beispiel 1 beschriebene Apparatur verwendet. Die aus der Ableitung 10 entweichenden Reaktionsprodukte werden durch nacheinandergeschaltete Waschflaschen zunächst in 20 gew%ige Natronlauge, dann in konzentrierte Schwefelsäure und anschließend durch eine mit Trockeneis gekühlte Kühlfalle geleitet.
Zur Herstellung des Anolyten werden 98 g (0,67 Mol) 3-H-Perfluorpropansäure (Formel II, X = H, p = 2) mit einer Lösung von 26,8 g (0,67 Mol) Natriumhydroxid in 100 g Wasser gemischt und anschließend mit 20 g Acetonitril und mit 6 g Brom versetzt. Der Katholyt besteht aus 25 gew%iger Schwefelsäure.

Die Elektrolyse erfolgt mit einer Rührgeschwindigkeit des Magnetstabes von 500 Upm bei einer Temperatur von 10°C und einer Anfangsstromstärke von 3 Ampere. Nach einem Ladungsdurchgang von 0,67 Faraday wird die Stromstärke auf 2 Ampere erniedrigt. Die gesamte Ladungsmenge beträgt 0,90 Faraday. Die Zellspannung liegt zwischen 12 und 14 Volt.
Im Verlauf der Elektrolyse werden weitere 60 g Brom in 12 Portionen dem Anolyten zugesetzt. Nach der Elektrolyse erwärmt man den Anolyten auf 40°C und treibt das restliche Produkt mit einem schwachen Stickstoffstrom über.
Das in der Kühlfalle gebildete farblose Kondensat (80,2 g) enthält nach gaschromatographischer Analyse 94,7 % 1-Brom-1,1,2,2-tetrafluorethan (Formel I, X = H, p = 2) und 4,7 % 1,1,2,2,3,3,4,4-Octafluorbutan, 0,3 % Acetonitril und 0,3 % einer unbekannten Verbindung. Die daraus berechnete Produktausbeute beträgt 63 %; die Stromausbeute beträgt 46 %.

### Beispiel 4

Für das Beispiel 4 wird die in Beispiel 1 beschriebene Apparatur verwendet.
Zur Herstellung des Anolyten werden 123 g (0,50 Mol) 5-H-Perfluorpentansäure (Formel II, X = H, p = 4) mit einer Lösung von 28 g (0,50 Mol) Kaliumhydroxid in 150 g Wasser vermischt und anschließend mit 50 g Acetonitril und 6 g Brom versetzt. Der Katholyt besteht aus 25 gew%iger Schwefelsäure.
Die Elektrolyse erfolgt bei einer Rührgeschwindigkeit des Magnetstabes von 500 Upm, bei einer Temperatur von 10°C und einer Stromstärke von 2,5 Ampere. Die Ladungsmenge beträgt 0,67 Faraday. Die Zellspannung liegt zwischen 12 und 24 Volt.
Im Verlauf der Elektrolyse werden weitere 50 g Brom in 10 Portionen dem Anolyten zugesetzt.

Nach der Elektrolyse wird die untere Phase des Anolyten abgetrennt und nacheinander 2 mal mit eiskalter 1 gew%iger Natronlauge, mit konzentrierter Schwefelsäure und Wasser gewaschen. Das erhaltene Rohprodukt (96 g) besteht nach gaschromatographischer Analyse aus 97 % 1-Brom-1,1,2,2,3,3,4,4-octafluorbutan (Formel I, X = H, p = 4). Durch Destillation erhält man ein Produkt, das bei 66 bis 67°C siedet. Die Produktausbeute beträgt 66 %, die Stromausbeute beträgt 55 %.

### Beispiel 5

Das Beispiel 5 wird in einer Anordnung mit einer geteilten Durchflußzelle mit parallel angeordneten plattenförmigen Elektroden von je 200 cm² Elektrodenfläche durchgeführt, wie in Fig. 2 schematisch dargestellt. Die Anode besteht aus einem Platinblech. Als Kathode dient eine Platte aus Graphit. Die Zelle wird durch eine Kationentauschermembran (Nafion ® 427) geteilt.
Der Anolytkreislauf besteht aus dem mit einer Kühlschlange versehenen Anolytbehälter 1, der Anolytpumpe 2 und dem Anodenraum 3 der Elektrolysezelle. Der Anolytbehälter 1 dient zur Abtrennung der gasförmigen Reaktionsprodukte, die über die Gasableitung 4 entweichen. Die Ausschleusung der flüssigen Reaktionsprodukte erfolgt über die Entnahmeleitung 5. Der Katholytkreislauf wird aus dem Katholytbehälter 1', der Katholytpumpe 2' und dem Kathodenraum 3' der Elektrolysezelle gebildet. Durch die Gasableitung 4' entweicht der bei der Elektrolyse gebildete Wasserstoff. Über die Entnahmeleitung 5' wird die kathodisch gebildete Alkalihydroxid-Lösung entnommen. An die Gasableitung 4 des Anolytkreislaufes wird eine mit Trockeneis gekühlte Falle angeschlossen.
Die Beschickung des Anolytkreislaufes erfolgt mit einer Lösung von 743 g (2,25 Mol) Perfluor-2-methyl-3-oxahexansäure (Formel IIa, q = 0; Reinheit ca. 98 %), die mit 1600 g 5,7 gew%iger wäßriger Natronlauge bis pH 5 neutralisiert und anschließend mit 500 g Acetonitril und 180 g (2,25 Mol) Brom versetzt wird.
Der Katholytkreislauf wird mit 300 g 1 gew%iger wäßriger Natronlauge beschickt. Die Umpumpgeschwindigkeit des Anolyten beträgt 200 l/h, diejenige des Katholyten 50 l/h.
Die Elektrolyse wird bei einer Temperatur von 25 - 30°C und einer Stromstärke von 30 Ampere bis zu einem Ladungsdurchgang von 2,92 Faraday durchgeführt; die erforderliche Zellspannung steigt dabei von anfänglich 7 Volt auf etwa 13 Volt an. Im Verlauf der Elektrolyse werden weitere 180 g Brom kontinuierlich zugetropft. Durch Zugabe von 5,7 gew%iger Natronlauge wird der pH-Wert des Anolyten während der Elektrolyse zwischen 3 und 3,5 gehalten. Das sich aus dem Anolyten als spezifisch schwerere Phase abscheidende Rohprodukt sowie das in der Kühlfalle aufgefangene Produkt werden zusammen mit 5 gew%iger wäßriger Natronlauge gewaschen. Man erhält 692 g Rohprodukt, das folgende, gaschromatografisch ermittelte Zusammensetzung aufweist (Angaben in Flächen-%):
97,8 % Perfluor-2-brom-3-oxahexan (Formel Ia; q = 0)
1,3 % Dimeres Produkt
0,6 % Perfluor-2-brom-5-methyl-3,6-dioxanonan (Formel Ia; q = 1)
Daraus errechnet sich eine Produktausbeute von 82,4 %, bezogen auf die eingesetzte Säure und eine Stromausbeute von 63,3 %.

### Beispiel 6

Für das Beispiel 6 wird die im Beispiel 5 beschriebene Apparatur benutzt.
Der Anolytkreislauf enthält 992 g (2,00 Mol) Perfluor-2,5-dimethyl-3,6-dioxanonansäure (Formel IIa, q = 1), die mit 1200 g 6,7 gew%iger wäßriger Natronlauge auf pH 5 - 6 neutralisiert ist, 500 g Acetonitril sowie 120 g (1.50 Mol) Brom. Der Katholytkreislauf besteht aus 300 g 1 gew%iger wäßriger Natronlauge. Die Umpumpgeschwindigkeit des Anolyten beträgt 200 l/h, diejenige des Katholyten beträgt 50 l/h. Bei einer Stromstärke von 30 Ampere und einer Temperatur von 30°C elektrolysiert man bis zu einem Ladungsdurchgang von 2,4 Faraday bei einer Zellspannung von 7,5 - 12,5 Volt. Im Verlauf der ersten Hälfte der Elektrolyse werden weitere 120 g Brom in den Anolyten kontinuierlich eindosiert. Während der Elektrolyse wird der pH-Wert des Anolyten durch Zugabe von 6,7 gew%iger wäßriger Natronlauge zwischen 3 und 4 gehalten. Das Produkt wird als spezifisch schwerere Phase aus dem Anolyten über die Entnahmeleitung 5 abgezogen. Nach Waschen mit 5 gew%iger wäßriger Natronlauge erhält man 974 g Rohprodukt, das folgende, gaschromatografisch ermittelte Zusammensetzung aufweist (Angaben in Flächen-%):
97,1 % Perfluor-2-brom-5-methyl-3,6-dioxanonan (Formel Ia, q = 1)
2,6 % Dimeres Produkt
0,3 % Perfluor-2-brom-3-oxahexan (Formel Ia, q = 0)
Daraus errechnet sich eine Produktausbeute von 89 %, bezogen auf die eingesetzte Säure, und eine Stromausbeute von 74 %.

### Beispiel 7

Für das Beispiel 7 wird die im Beispiel 5 beschriebene Apparatur benutzt.
Der Anolytkreislauf wird mit einer Lösung beschickt, die folgendermaßen hergestellt wurde: 1324 g (2,0 Mol) Perfluor-2,5,8-trimethyl-3,6,9-trioxadodecansäure (Formel IIa, q = 2) werden mit 1200 g 6,7 gew%iger wäßriger Natronlauge vermischt und mit 500 g Acetonitril sowie 120 g (1,5 Mol) Brom versetzt. Der Katholytkreislauf besteht aus 300 g 1 gew%iger wäßriger Natronlauge. Die Umpumpgeschwindigkeit des Anolyten beträgt 200 l/h, diejenige des Katholyten 50 l/h.
Bei einer Elektrolysetemperatur von 25 - 30°C und einer Stromstärke von 20 Ampere wird bis zu einem Ladungsdurchgang von 2,4 Faraday bei einer Zellspannung von 7 - 14 Volt elektrolysiert. Im Verlauf der Elektrolyse werden weitere 160 g Brom in den Anolyten kontinuierlich eindosiert. Nach Beendigung der Elektrolyse trennt man die spezifisch schwerere Phase vom Anolyten ab und wäscht sie mit 5 gew%iger wäßriger Natronlauge. Als Rückstand erhält man 1126 g Rohprodukt, das folgende, gaschromatografisch ermittelte Zusammensetzung aufweist (Angaben in Flächen-%):
96,5 % Perfluor-2-brom-5,8-dimethyl-3,6,9-trioxadodecan
Formel Ia, q = 2)
1,5 % Dimeres Produkt
0,8 % Perfluor-2-brom-5-methyl-3,6-dioxanonan (Formel Ia, q = 1)
Das entspricht einer Produktausbeute von 78 %, bezogen auf die eingesetzte Säure, und einer Stromausbeute von 73 %.

## Patentansprüche

1. Verfahren zur Herstellung von perfluorierten Bromalkanen der allgemeinen Formel
X-(CF₂)p-Br (I),
worin X Wasserstoff oder Fluor bedeutet und p für eine ganze Zahl von 2 bis 10 steht,
oder von Ethergruppen enthaltenden perfluorierten Bromalkanen der allgemeinen Formel 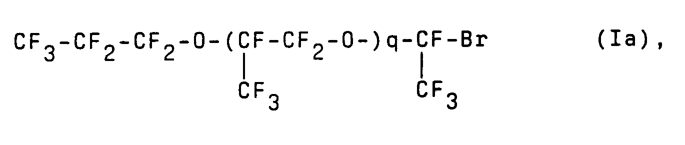 worin
q für Null oder eine ganze Zahl von 1 bis 4 steht,
dadurch gekennzeichnet, daß man eine perfluorierte Alkancarbonsäure der allgemeinen Formel
X-(CF₂)p-COOH (II),
worin X Wasserstoff oder Fluor bedeutet und p für eine ganze Zahl von 2 bis 10 steht,
oder eine Ethergruppen enthaltende Alkancarbonsäure der allgemeinen Formel 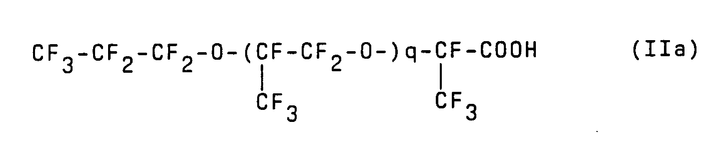 worin
q für Null oder eine ganze Zahl von 1 bis 4 steht,
in einem wäßrigen Elektrolyten in Gegenwart von Brom und einem aliphatischen Nitril einer elektrolytischen Decarboxylierung unterwirft.

2. Verfahren nach Anspruch 1, dadurch gekennzeichnet, daß man ein aliphatisches Nitril mit 1 bis 6 C-Atomen verwendet.

3. Verfahren nach Anspruch 1 oder 2, dadurch gekennzeichnet, daß man als aliphatisches Nitril Acetonitril, Propionitril oder Isobutyronitril verwendet.

4. Verfahren nach einem der Ansprüche 1 bis 3, dadurch gekennzeichnet, daß man als wäßrigen Elektrolyten ein Gemisch aus 1 bis 40 Gew% Nitril
1 bis 70 Gew% Alkancarbonsäure der Formel II oder IIa
5 bis 100 mol% Alkalihydroxid, berechnet auf die Alkancarbonsäure
50 bis 200 mol% Brom, berechnet auf die Alkancarbonsäure
Rest Wasser elektrolysiert.

5. Verfahren nach einem der Ansprüche 1 bis 4, dadurch gekennzeichnet, daß man als Alkancarbonsäuren
α) Omega-H-Perfluoralkansäuren der Formel II mit X = H: 3-H-Perfluorpropionsäure, 5-H-Perfluorpentansäure, 7-H-Perfluorheptansäure, 9-H-Perfluornonansäure;
β) Perfluoralkansäuren der Formel II mit X= F: Perfluorpentansäure, Perfluorhexansäure, Perfluorheptansäure, Perfluoroctansäure, Perfluornonansäure, Perfluordecansäure oder Perfluorundekansäure;
γ) Perfluorethercarbonsäuren der Formel IIa: Perfluor-2-methyl-3-oxahexansäure, Perfluor-2,5-dimethyl-3,6-dioxanonansäure, Perfluor-2,5,8-trimethyl-3,6,9-trioxadodecansäure oder Perfluor-2,5,8,11-tetramethyl-3,6,9,12-tetraoxapentadecansäure verwendet.

6. Verfahren nach einem der Ansprüche 1 bis 5, dadurch gekennzeichnet, daß man die Alkancarbonsäuren in neutralisierter oder teilweise neutralisierter Form einsetzt.

7. Verfahren nach Anspruch 6, dadurch gekennzeichnet, daß man als Alkalihydroxid zur Neutralisation oder Teilneutralisation der Alkancarbonsäure Natriumhydroxid, Kaliumhydroxid oder Tetraalkylammoniumhydroxid einsetzt.

8. Verfahren nach einem der Ansprüche 1 bis 7, dadurch gekennzeichnet, daß man während der elektrolytischen Decarboxylierung Alkancarbonsäure in nichtneutralisierter Form in dem Maße nachdosiert, wie diese bei der elektrolytischen Decarboxylierung verbraucht wird.

9. Verfahren nach einem der Ansprüche 1 bis 8, dadurch gekennzeichnet, daß man das Brom portionsweise oder kontinuierlich während der elektrolytischen Decarboxylierung dem Elektrolyten zugibt.

10. Verfahren nach einem der Ansprüche 1 bis 9, dadurch gekennzeichnet, daß man eine Stromdichte von 20 bis 500, insbesondere von 50 bis 250 mA/cm² einstellt.

11. Verfahren nach einem der Ansprüche 1 bis 10, dadurch gekennzeichnet, daß man die elektrolytische Decarboxylierung bei Temperaturen zwischen -10°C und 50°C durchführt.

12. Verfahren nach einem der Ansprüche 1 bis 11, dadurch gekennzeichnet, daß man die elektrolytische Decarboxylierung in ungeteilten Becherglaszellen oder Durchflußzellen durchführt.

13. Verfahren nach einem der Ansprüche 1 bis 11, dadurch gekennzeichnet, daß man die elektrolytische Decarboxylierung in einer geteilten Elektrolysezelle durchführt, wobei der Anolyt- und Kathodenraum durch eine ionenselektive Membran oder ein Diaphragma voneinander getrennt sind.

14. Verfahren nach Anspruch 13, dadurch gekennzeichnet, daß man die Alkancarbonsäure und das Brom in den Anolytraum gibt.

## Claims

1. A process for preparing perfluorinated bromoalkanes of the formula
X-(CF₂)ₚ-Br (I),
in which X is hydrogen or fluorine and p is an integer from 2 to 10,
or perfluorinated bromoalkanes which contain ether groups, of the formula 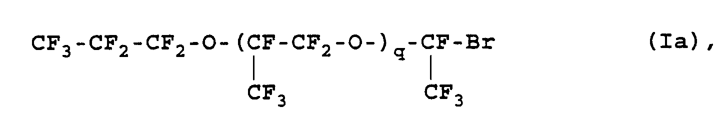 in which
q is zero or an integer from 1 to 4,
which comprises subjecting a perfluorinated alkanecarboxylic acid of the formula
X-(CF₂)ₚ-COOH (II),
in which X is hydrogen or fluorine and p is an integer from 2 to 10,
or an alkanecarboxylic acid which contains ether groups, of the formula 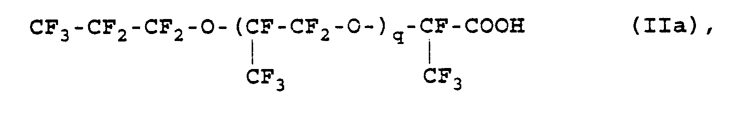 in which q is zero or an integer from 1 to 4, to electrolytic decarboxylation in an aqueous electrolyte in the presence of bromine and an aliphatic nitrile.

2. The process as claimed in claim 1, wherein an aliphatic nitrile having from 1 to 6 carbon atoms is used.

3. The process as claimed in claim 1 or 2, wherein the aliphatic nitrile used is acetonitrile, propionitrile or isobutyronitrile.

4. The process as claimed in any one of claims 1 to 3, wherein the aqueous electrolyte electrolyzed is a mixture comprising
from 1 to 40% by weight of nitrile
from 1 to 70% by weight of alkanecarboxylic acid of the formula II or IIa
from 5 to 100 mol% of alkali metal hydroxide, based on the alkanecarboxylic acid
from 50 to 200 mol% of bromine, based on the alkanecarboxylic acid
the remainder being water.

5. The process as claimed in any one of claims 1 to 4, wherein the alkanecarboxylic acids used are
α) omega-H-perfluoroalkanoic acids of the formula II with X = H:
3-H-perfluoropropionic acid, 5-H-perfluoropentanoic acid, 7-H-perfluoroheptanoic acid, 9-H-perfluorononanoic acid;
β) perfluoroalkanoic acids of the formula II with X = F:
perfluoropentanoic acid, perfluorohexanoic acid, perfluoroheptanoic acid, perfluorooctanoic acid, perfluorononanoic acid, perfluorodecanoic acid or perfluoroundecanoic acid;
γ) perfluoroethercarboxylic acids of the formula IIa:
perfluoro-2-methyl-3-oxahexanoic acid, perfluoro2,5-dimethyl-3,6-dioxanonanoic acid, perfluoro-2, 5, 8-trimethyl-3, 6, 9-trioxadodecanoic acid or perfluoro-2,5,8,11-tetramethyl-3, 6, 9, 12-tetraoxapentadecanoic acid.

6. The process as claimed in any one of claims 1 to 5, wherein the alkanecarboxylic acids are employed in neutralized or partially neutralized form.

7. The process as claimed in claim 6, wherein the alkali metal hydroxide employed for neutralization or part-neutralization of the alkanecarboxylic acid is sodium hydroxide, potassium hydroxide or tetraalkylammonium hydroxide.

8. The process as claimed in any one of claims 1 to 7, wherein during the electrolytic decarboxylation more alkanecarboxylic acid in non-neutralized form is metered in to the extent in which it is consumed in the electrolytic decarboxylation.

9. The process as claimed in any one of claims 1 to 8, wherein the bromine is added to the electrolyte in portions or continuously during the electrolytic decarboxylation.

10. The process as claimed in any one of claims 1 to 9, wherein a current density of 20 to 500, in particular of 50 to 250, mA/cm² is set.

11. The process as claimed in any one of claims 1 to 10, wherein the electrolytic decarboxylation is carried out at temperatures between -10°C and 50°C.

12. The process as claimed in any one of claims 1 to 11, wherein the electrolytic decarboxylation is carried out in undivided beaker cells or flow cells.

13. The process as claimed in any one of claims 1 to 11, wherein the electrolytic decarboxylation is carried out in a divided electrolytic cell, the anolyte compartment and cathode compartment being separated from one another by an ion-selective membrane or a diaphragm.

14. The process as claimed in claim 13, wherein the alkanecarboxylic acid and the bromine are placed into the anolyte compartment.

## Revendications

1. Procédé de préparation de bromoalcanes perfluorés de formule générale
X-(CF₂)p-Br (I)
dans laquelle X représente l'hydrogène ou le fluor et p est un nombre entier allant de 2 à 10,
ou de bromoalcanes perfluorés contenant des groupes éthers, de formule générale 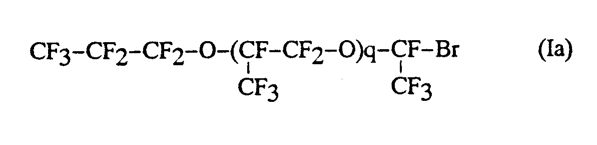 dans laquelle q est égal à 0 ou est un nombre entier allant de 1 à 4,
caractérisée en ce que l'on soumet à une décarboxylation électrolytique un acide alcanoïque perfluoré de formule générale
X-(CF₂)p-COOH (II)
dans laquelle X représente l'hydrogène ou le fluor et p est un nombre entier allant de 2 à 10,
ou un acide alcanoïque contenant des groupes éthers, de formule générale 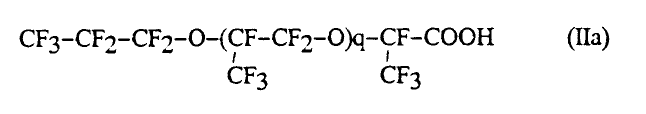 dans laquelle
q est égal à 0 ou est un nombre entier allant de 1 à 4,
dans un électrolyte aqueux en présence de brome et d'un nitrile aliphatique.

2. Procédé selon la revendication 1, caractérisé en ce que l'on utilise un nitrile aliphatique en C₁-C₆.

3. Procédé selon la revendication 1 ou 2, caractérisé en ce que le nitrile aliphatique utilisé est l'acétonitrile, le propionitrile ou l'isobutyronitrile.

4. Procédé selon une des revendications 1 à 3, caractérisé en ce que l'on utilise en tant qu'électrolyte aqueux un mélange de
1 à 40 % en poids de nitrile,
1 à 70 % en poids d'un acide alcanoïque de formule II ou IIa,
5 à 100 mol % d'un hydroxyde alcalin, par rapport à l'acide alcanoïque,
50 à 200 mol % de brome, par rapport à l'acide alcanoïque, le solde consistant en eau.

5. Procédé selon une des revendications 1 à 4, caractérisé en ce que l'on met en oeuvre en tant qu'acides alcanoïques
α) des acides Ω-H-perfluoralcanoïques de formule II dans laquelle X = H :
l'acide 3-H-perfluoropropionique, l'acide 5-H-perfluoropentanoïque, l'acide 7-H-perfluoroheptanoïque, l'acide 9-H-perfluorononanoïque;
β) des acides pefluoroalcanoïques de formule II dans laquelle X=F:
l'acide perfluoropentanoïque, l'acide perfluorohexanoïque, l'acide perfluoroheptanoïque, l'acide perfluorooctanoïque, l'acide perfluorononanoïque, l'acide perfluorodécanoïque ou l'acide perfluoroundécanoïque;
γ) des acides perfluoréthercarboxyliques de formule IIa :
l'acide perfluoro-2-méthyl-3-oxahexanoïque, l'acide perfluoro-2,5-diméthyl-3,6-dioxanonanoïque, l'acide perfluoro-2,5,8-triméthyl-3,6,9-trioxadodécanoïque ou l'acide perfluoro-2,5,8,11-tétraméthyl-3,6,9,12-tétraoxapentadécanoïque.

6. Procédé selon une des revendications 1 à 5, caractérisé en ce que l'on met en oeuvre les acides alcanoïques à l'état neutralisé en totalité ou en partie.

7. Procédé selon la revendication 6, caractérisé en ce que l'on utilise en tant qu'hydroxyde alcalin pour la neutralisation totale ou partielle de l'acide alcanoïque, l'hydroxyde de sodium, l'hydroxyde de potassium ou un hydroxyde de tétraalkylammonium.

8. Procédé selon une des revendications 1 à 7, caractérisé en ce que, durant la décarboxylation électrolytique on ajoute de l'acide alcanoïque à l'état non neutralisé au fur et à mesure de sa consommation dans la décarboxylation électrolytique.

9. Procédé selon une des revendications 1 à 8, caractérisé en ce que l'on ajoute du brome par portions ou en continu à l'électrolyte au cours de la décarboxylation électrolytique.

10. Procédé selon une des revendications 1 à 9, caractérisé en ce que l'on règle à une densité de courant de 20 à 500, plus spécialement de 50 à 250 mA/cm².

11. Procédé selon une des revendications 1 à 10, caractérisé en ce que l'on réalise la décarboxylation électrolytique à des températures allant de -10°C à 50°C.

12. Procédé selon une des revendications 1 à 11, caractérisé en ce que l'on réalise la décarboxylation électrolytique dans des cellules en verre non compartimentées du type bécher ou dans des cellules à écoulement continu.

13. Procédé selon une des revendications 1 à 11, caractérisé en ce que l'on réalise la décarboxylation électrolytique dans une cellule d'électrolyse compartimentée dans laquelle le compartiment anodique et le compartiment cathodique sont séparés l'un de l'autre par une membrane à sélectivité ionique ou par un diaphragme.

14. Procédé selon la revendication 13, caractérisé en ce que l'on introduit l'acide alcanoïque et le brome dans le compartiment anodique.
